# FASCICULE DE BREVET EUROPEEN

(11) **EP 1 183 157 B1**
(45) Date de publication et mention de la délivrance du brevet: **17.09.2003**
(21) Numéro de dépôt: 99973834.7
(22) Date de dépôt: 25.06.1999
(51) Int. Cl.: B44C 1/175, B65D 81/32

(54) **DISPOSITIF D'APPLICATION D'UN TATOUAGE TEMPORAIRE ADHESIF CUTANE ET D'ADMINISTRATION D'UN PRODUIT PHARMACEUTIQUE ET/OU COSMETIQUE**
VORRICHTUNG ZUM AUFBRINGEN EINER KLEBETÄTOWIERUNG UND ZUM HANDHABEN VON PHARMAZEUTISCHEN ODER KOSMETISCHEN PRODUKTEN
DEVICE FOR APPLYING AN ADHESIVE SKIN TATTOO AND FOR ADMINISTERING A PHARMACEUTICAL AND/OR COSMETIC PRODUCT

(30) Priorité: 04.05.1999 FR 9905660
(43) Date de publication de la demande: 06.03.2002
(73) Titulaire: Martel, Stephane Christopher, 92250 La Garenne Colombes (FR); Bochenek, Valerie Frederique, 80200 Doingt (FR)
(72) Inventeur: Martel, Stephane Christopher, 92250 La Garenne Colombes (FR); Bochenek, Valerie Frederique, 80200 Doingt (FR)
(74) Mandataire: Goulard, Sophie
(86) Numéro de dépôt international: FR9901538
(87) Numéro de publication internationale: WO00066371

(56) Documents cités:
- EP-A- 0 294 189
- EP-A- 0 737 463
- WO-A-98/45127
- CH-A- 626 542
- US-A- 4 430 013

## Description

La présente invention a pour objet un dispositif d'application de tatouage temporaire cutané, permettant éventuellement l'administration d'un produit médicamenteux et/ou cosmétique par voie cutanée.

Le dispositif selon l'invention s'applique notamment à des produits ludiques, promotionnels, événementiels, publicitaires, commerciaux, cosmétiques et pharmaceutiques.

Le document EP 294 189 décrit un dispositif d'application à l'aide d'un tampon, d'un fluide contenu dans une poche étanche adaptée pour se rompre sous la pression.

Le brevet américain US 5578353 décrit un dispositif de décalcomanie d'un ticket portant une encre, à l'aide d'une éponge mouillée ou d'une pulvérisation appliquée sur la peau. Ce dispositif nécessite, soit l'utilisation d'une éponge mouillée, soit d'un moyen équivalent, ce qui complique l'utilisation.

La demande de brevet allemand DE 19606839 décrit un dispositif comportant un support auxiliaire, une couche d'adhésif de contact et une couche de transfert entre le support et la couche d'adhésif, ladite couche de transfert contenant un liant, qui adhère plus fortement à la couche adhésive qu'au support, la couche de transfert et/ou la couche adhésive comportant, d'une part de fines particules creuses contenant de l'eau et, d'autre part, un alcool solide soluble dans l'eau, de façon qu'une pression exercée sur le support, provoque l'éclatement des microparticules pour appliquer ladite couche de transfert sur une surface. Un tel dispositif nécessite par conséquent, de fabriquer des microsphères dans une couche de polymère opaque, ladite couche de polymère opaque étant destinée à être transférée sur la surface, ce qui complique la fabrication et augmente le coût de production.

La demande Internationale WO 98/45127 décrit un dispositif d'application de tatouage temporaire, sur n'importe quel support, comprenant d'une part, une réserve, définie par une enveloppe en matière souple déformable, ladite réserve étant pourvue d'au moins une zone d'affaiblissement, et d'autre part, un tampon applicateur portant une encre pour tatouage temporaire, de maniére à générer l'humidification dudit tampon applicateur lorsqu'une pression, exercée sur 1a réserve, provoque son éclatement au niveau de la zone d'affaiblissement. Ce dispositif possède une pré-ouverture de petite dimension, ce qui rend nécessaire une durée d'application longue pour permettre une diffusion adéquate du liquide et une bonne imprégnation du tampon applicateur.

Les Inventeurs se sont donné pour but de pourvoir à un dispositif d'application de tatouage temporaire adhésif cutané, qui soit simple à utiliser, peu coûteux à fabriquer et qui permette une imprégnation rapide et très homogène du tampon applicateur.

La présente invention a en conséquence pour objet un dispositif d'application de tatouage temporaire adhésif comprenant un support souple ayant deux faces principales opposées, l'une des faces présentant une ouverture, recouverte d'un tampon applicateur muni d'un tatouage adhésif cutané temporaire, alors que l'autre face supporte une réserve de liquide déformable, dans lequel:
- une cavité intermédiaire étanche est ménagée au-dessus du tampon applicateur,
- la réserve de liquide, située en dehors de ladite ouverture, est sensiblement de forme rectangulaire, ovale ou oblongue et
- la réserve de liquide et la cavité intermédiaire ont pour but de communiquer entre elles par un passage, ledit passage s'étendant sur une largeur correspondant au plus grand côté de-la réserve et comprenant une zone de contrainte s'étendant également sur une largeur correspondant au plus grand côté de la réserve et un moyen formant étanchéité, ledit moyen étant destiné à se rompre ou à se détacher pour libérer le fluide contenu dans la réserve en direction du tampon applicateur via la cavité intermédiaire, lorsqu'une pression suffisante est exercée sur la réserve de liquide, de manière à humidifier le tampon applicateur muni sur sa surface externe d'un tatouage adhésif cutané temporaire.

Ladite zone de contrainte est une zone non hermétique qui a pour fonction de canaliser le liquide contenu dans la réserve en direction du tampon applicateur *via* la cavité intermédiaire lorsqu'une pression suffisante est exercée sur la réserve de liquide.

Dans un mode avantageux de réalisation du dispositif selon la l'invention, la zone de contrainte communique avec la réserve et forme l'ouverture de sortie de ladite réserve lorsqu'une pression est exercée sur cette dernière.

Dans un autre mode avantageux de réalisation du dispositif selon l'invention, le support est formé par au moins deux pellicules hermétiquement liées l'une à l'autre à leur périphérie par un premier moyen de liaison définitif et par un deuxième moyen de liaison non définitif, la pellicule supérieure présentant deux embossages permettant de définir, avec la pellicule inférieure, la réserve de liquide et la cavité intermédiaire étanche ; avantageusement, les pellicules sont hermétiquement liées l'une à l'autre par collage ou thermosoudage, ledit premier moyen étant un collage ou un thermosoudage définitif et ledit deuxième moyen étant un collage ou un thermosoudage fragilisé.

Dans un autre mode de réalisation avantageux dudit dispositif l'ouverture et la cavité intermédiaire étanche ont sensiblement la même dimension que le tampon applicateur.

Dans un autre mode avantageux de réalisation dudit dispositif, le tampon applicateur, hermétiquement lié au support, sur toute sa périphérie, par un moyen de liaison, ledit moyen de liaison étant notamment un cordon de colle ou une thermosoudure définitive, recouvre entièrement l'ouverture.

Dans un autre mode de réalisation du dispositif selon l'invention, le tampon applicateur est réalisé dans une matière absorbante, notamment choisie dans le groupe constitué par du papier absorbant, et des matières textiles cotonneuses ou spongieuses.

Selon un autre mode de réalisation avantageux du dispositif selon l'invention, les pellicules sont, indépendamment l'une de l'autre, en matière plastique ou analogue, composite ou métallique. Avantageusement, la pellicule inférieure est en matière composite comprenant de l'aluminium, la pellicule supérieure en copolymère transparent et le tampon applicateur est constitué par du papier buvard.

Dans une autre variante particulièrement avantageuse du dispositif selon l'invention, le passage comprend comme moyen formant étanchéité une soudure fragilisée susceptible de se rompre ou de se détacher, lorsqu'une pression suffisante est exercée sur 1a réserve de liquide et comme zone de contrainte, une zone non soudée.

Il va de soi que dans le cadre de la présente invention, ledit moyen d'étanchéité, notamment la soudure fragilisée, ne se rompra ou ne se détachera que lorsqu'une pression suffisamment élevée sera exercée sur la réserve, ceci afin de garantir l'intégrité dudit moyen, lors du transport et du stockage du dispositif.

Le dispositif selon l'invention peut comprendre en outre un film protecteur escamotable, apte à recouvrir complètement le tampon applicateur et à protéger le tatouage adhésif cutané temporaire de divers éléments extérieurs pouvant le contaminer ou l'endommager. Ce film protecteur peut éventuellement être muni, sur sa couche externe, d'une couche de matière adhésive pelable, qui permet le retrait du tatouage temporaire de la peau et son transfert sur un autre support, notamment un collecteur.

Avec le dispositif selon la présente invention, lorsqu'une pression suffisante est exercée sur la réserve de liquide, la zone de contrainte permet la rupture ou le détachement du moyen d'étanchéité, autorisant la libération du fluide contenu dans ladite réserve et son passage vers la cavité intermédiaire d'où il diffuse de manière homogène et rapide sur le tampon applicateur, ce qui entraîne son humidification et le transfert instantané du tatouage temporaire adhésif sur la peau.

Du fait de la relativement grande dimension de la zone de contrainte, la diffusion est beaucoup plus homogène et rapide qu'avec les dispositifs connus de l'état antérieur de la technique.

De plus le dispositif selon l'invention permet de mieux maîtriser le processus de diffusion, évitant ainsi les pertes de liquide.

D'autres caractéristiques et avantages de l'invention apparaissent dans la suite de la description détaillée qui suit et fait référence aux figures annexées, dans lesquelles :
- la figure 1 est une vue schématique en coupe transversale d'un mode de réalisation avantageux du dispositif selon l'invention,
- la figure 2 est une vue schématique en coupe transversale du mode de réalisation représenté à la figure 1, durant son utilisation,
- la figure 3 représente une vue de dessus du dispositif selon le mode de réalisation représenté à la figure 1 (variante 2) et d'un deuxième mode de réalisation (variante 1).

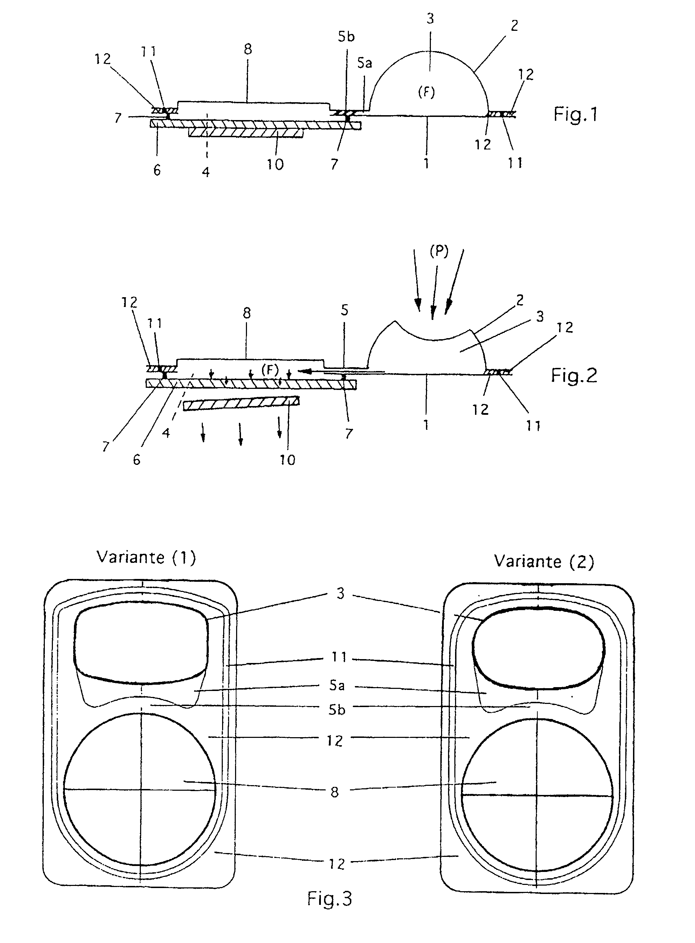

On va maintenant décrire un premier mode de réalisation de l'invention, en référence aux figures 1 à 3.

Le dispositif d'application de tatouage temporaire adhésif cutané, comprend un support souple ayant deux faces principales opposées, ledit support étant formé par deux pellicules (1, 2) hermétiquement liées l'une à l'autre à leur périphérie, par exemple par une thermosoudure définitive (11) et une thermosoudure fragilisée non définitive (12).

La pellicule (1) présente sur une de ses faces, une ouverture (4), recouverte d'un tampon applicateur (6) muni d'un tatouage adhésif cutané temporaire (10). Le tampon applicateur (6) est lié à la pellicule (1) par une thermosoudure (7).

L'autre face de la pellicule (1) supporte une réserve de liquide déformable (3), sensiblement rectangulaire, ovale ou oblongue et une cavité intermédiaire étanche (8), aménagée au-dessus du tampon applicateur (6).

Ladite réserve (3) et ladite cavité (8) sont définies entre la pellicule (1), réalisée par exemple en un matériau composite comprenant de l'aluminium, et deux embossages formés dans une pellicule (2), réalisée par exemple dans un copolymère.

La réserve de liquide (3) et la cavité intermédiaire (8) ont pour but de communiquer entre elles par un passage (5), s'étendant sur une largeur correspondant au plus grand côté de la réserve (3) et comprenant une zone de contrainte (Sa) s'étendant également sur une largeur correspondant au plus grand côté de la réserve (3) et un moyen formant étanchéité (5b), ledit moyen étant destiné à se rompre ou à se détacher pour libérer le fluide contenu dans la réserve (3) en direction du tampon applicateur (6).

Ainsi lorsqu'une pression manuelle (P) est exercée sur la réserve (3), elle provoque l'ouverture de la zone de passage (5), par rupture ou détachement du moyen d'étanchéité (5b), lequel peut être, par exemple, une soudure fragilisée.

Cette rupture ou ce détachement permet le passage du fluide (F) dans la cavité intermédiaire (8), la diffusion dudit fluide sur le tampon applicateur (6) et le transfert du tatouage adhésif temporaire (10).

Le support du dispositif selon l'invention peut également porter, par exemple sur la surface portant la réserve (3), au dessus de l'ouverture (4), un message publicitaire ou un information sur le produit contenu dans la réserve (3), notamment lorsque ce produit est un médicament.

Bien que l'invention ait été décrite en relation avec des modes de réalisation particuliers, il est bien entendu qu'elle n'y est nullement limitée et qu'elle comprend tous les équivalents techniques des moyens décrits ainsi que leurs différentes combinaisons entrant dans le cadre de la présente invention.

## Revendications

1. Dispositif d'application de tatouage temporaire adhésif comprenant un support souple ayant deux faces principales opposées, l'une des faces présentant une ouverture (4), recouverte d'un tampon applicateur (6) muni d'un tatouage adhésif cutané temporaire (10), alors que l'autre face supporte une réserve de liquide déformable (3), dans lequel:
- une cavité intermédiaire étanche (8) est ménagée au-dessus du tampon applicateur (6),
- la réserve de liquide (3), située en dehors de ladite ouverture (4), est sensiblement de forme rectangulaire, ovale ou oblongue et
- la réserve de liquide (3) et la cavité intermédiaire (8) ont pour but de communiquer entre elles par un passage (5), ledit passage s'étendant sur une largeur correspondant au plus grand côté de la réserve et comprenant une zone de contrainte (5a) s'étendant également sur une largeur correspondant au plus grand côté de la réserve et un moyen formant étanchéité (5b), ledit moyen étant destiné à se rompre ou à se détacher pour libérer le fluide contenu dans la réserve (3) en direction du tampon applicateur (6) *via* la cavité intermédiaire (8), lorsqu'une pression suffisante est exercée sur la réserve de liquide (3), de manière à humidifier le tampon applicateur (6) muni sur sa surface externe d'un tatouage adhésif cutané temporaire (10).

2. Dispositif selon la revendication 1, **caractérisé en ce que** la zone de contrainte (5a) communique avec la réserve (3) et forme l'ouverture de sortie de ladite réserve (3) lorsqu'une pression est exercée sur cette dernière.

3. Dispositif selon l'une quelconque des revendications 1 et 2, **caractérisé en ce que** le support est formé par au moins deux pellicules (1, 2) hermétiquement liées l'une à l'autre à leur périphérie, par un premier moyen de liaison définitif (11) et par un deuxième moyen de liaison non définitif (12), la pellicule (2) présentant deux embossages permettant de définir, avec la pellicule (1), la réserve de liquide (3) et la cavité intermédiaire étanche (8).

4. Dispositif selon la revendication 3, **caractérisé en ce que** les pellicules (1) et (2) sont hermétiquement liées l'une à l'autre par collage ou thermosoudage, le moyen (11) étant un collage ou un thermosoudage définitif et le moyen (12) étant un collage ou un thermosoudage fragilisé.

5. Dispositif selon l'une quelconque des revendications 1 à 4, **caractérisé en ce que** l'ouverture (4) et la cavité intermédiaire étanche (8) ont sensiblement la même dimension que le tampon applicateur (6).

6. Dispositif selon l'une quelconque des revendications 1 à 5, **caractérisé en ce que** le tampon applicateur (6), hermétiquement lié au support, sur toute sa périphérie, par un moyen de liaison (7), ledit moyen de liaison (7) étant un cordon de colle ou une thermosoudure définitive, recouvre entièrement l'ouverture (4).

7. Dispositif selon l'une quelconque des revendications 1 à 6, **caractérisé en ce que** le tampon applicateur est réalisé dans une matière absorbante choisie dans le groupe constitué par du papier absorbant et des matières textiles cotonneuses ou spongieuses.

8. Dispositif selon l'une quelconque des revendications 2 à 7, **caractérisé en ce que** les pellicules (1) et (2) sont, indépendamment l'une de l'autre, en matière plastique ou analogue, composite ou métallique.

9. Dispositif selon les revendications 7 et 8, **caractérisé en ce que** la pellicule (1) est une matière composite comprenant de l'aluminium, la pellicule (2) est un copolymère transparent et le tampon applicateur (6) est constitué par du papier buvard.

10. Dispositif selon l'une quelconque des revendications 1 à 9, **caractérisé en ce que** le passage (5) comprend comme moyen formant étanchéité (5b), une soudure fragilisée, susceptible de se rompre ou de se détacher, lorsqu'une pression suffisante est exercée sur la réserve de liquide (3), et comme zone de contrainte (5a), une zone non soudée.

11. Dispositif selon l'une quelconque des revendications 1 à 10, **caractérisé en ce qu'**il comprend un film protecteur escamotable, apte à recouvrir complètement le tampon applicateur et à protéger le tatouage adhésif cutané temporaire, ledit film protecteur pouvant éventuellement être muni, sur sa couche externe, d'une couche de matière adhésive pelable.

## Claims

1. Device for applying an adhesive temporary tattoo comprising a flexible support having two main opposite faces, one of the faces having an opening (4) covered with an applicator pad (6) provided with a skin adhesive temporary tattoo (10), while the other face supports a deformable liquid reserve (3), in which:
- an intermediate watertight hollow (8) is arranged above the applicator pad (6),
- the liquid reserve (3), located on the outside of said opening (4), has a more or less rectangular, oval or oblong shape, and
- the liquid reserve (3) and the intermediate hollow (8) are intended to communicate between themselves through a passage (5), said passage extending on a width corresponding to the largest side of the reserve and comprising a constrain region (5a) extending also on a width corresponding to the largest side of the reserve and a watertight forming mean (5b), said mean being designed to break or to become detached to release the fluid contained in the reserve (3) towards the applicator pad (6) *via* the intermediate hollow (8), when a sufficient pressure is exerted on the liquid reserve (3), in order to humidify the applicator pad (6) provided with, on its external surface, with a temporary skin adhesive tattoo (10).

2. Device according to claim 1, **characterized in that** constrain region (5a) communicates with the reserve (3) and forms the exit opening of said reserve (3) when a pressure is exerted on the reserve.

3. Device according to any one of claims 1 and 2, **characterized in that** the support is formed by at least two films (1, 2) which are hermetically bonded to each other in a peripheral region, by a first definitive bonding mean (11) and by a second temporary bonding mean (12), the film (2) having two embossments defining, with the film (1), the liquid reserve (3) and the intermediate watertight hollow (8).

4. Device according to claim 3, **characterized in that** the films (1) et (2) are hermetically bonded to each other by adhesive bonding or by heat sealing, the bonding mean (11) being an adhesive bonding or a definitive heat sealing and the bonding mean (12) being an adhesive bonding or a weakened heat sealing.

5. Device according to any one of claims 1 to 4, **characterized in that** the opening (4) and the intermediate watertight hollow (8) have approximately the same size than the applicator pad (6).

6. Device according to any one of claims 1 to 5, **characterized in that** the applicator pad (6), which is hermetically bonded to the support, all along its peripheral region, by a bonding mean (7), said bonding mean being an glue string or a definitive heat sealing, entirely covers the opening (4).

7. Device according to any one of claims 1 to 6, **characterized in that** the applicator pad is made from an absorbing material selected from the group consisting of absorbing paper and fluffy or spongy textile materials.

8. Device according to any one of claims 2 to 7, **characterized in that** the films (1) and (2) are made of, independently the one from the other, a plastic or the like, a composite or a metallic material.

9. Device according to claims 7 and 8, **characterized in that** the film (1) is made of a composite material comprising aluminium, the film (2) is a transparent polymer and the applicator pad (6) is made of blotting paper.

10. Device according to any one of claims 1 to 9, **characterized in that** the passage (5) comprises, as watertight forming mean (5b), a weakened welding, which is susceptible of being break or detached, when a sufficient pressure is exerted on the liquid reserve (3), and as constrain region (5a), a unsealed region.

11. Device according to any one of claims 1 to 10, **characterized in that** it comprises a removable protective film, which is able to entirely cover the applicator pad and to protect the skin adhesive temporary tattoo, said protective film being optionally provided, on its external layer, with a peelable adhesive material layer.

## Patentansprüche

1. Vorrichtung zum Aufbringen einer zeitlich begrenzt haftenden Tätowierung, umfassend einen flexiblen Träger mit zwei gegenüberliegenden Hauptseiten, wobei eine dieser Seiten eine mit einem Aufbringungskissen (6), welches mit einer zeitlich begrenzt haftenden kutanen Tätowierung ausgestattet ist, bedeckte Öffnung (4) aufweist, während die andere Seite einen deformierbaren Flüssigkeitsvorrat (3) trägt, wobei:
- eine dichte Zwischenkavität (8) oberhalb des Aufbringungskissens (6) angeordnet ist,
- der außerhalb der Öffnung (4) angeordnete Flüssigkeitsvorrat (3) im Wesentlichen eine rechteckige, ovale oder längliche Form aufweist, und
- der Flüssigkeitsvorrat (3) und die Zwischenkavität (8) ausgestaltet sind, um miteinander durch einen Gang (5) in Verbindung zu stehen, wobei sich der Gang auf einer Breite entsprechend der größten Seite des Vorrats erstreckt und eine Druckzone (5a) umfasst, welche sich ebenfalls auf einer der größten Seite des Vorrats entsprechenden Breite erstreckt und ein Abdichtungsmittel (5b) umfasst, wobei dieses Mittel ausgestaltet ist, beschädigt zu werden oder sich abzulösen, um die in dem Vorrat (3) enthaltene Flüssigkeit in Richtung des Aufbringungskissens (6) über die Zwischenkavität (8) in einer Weise freizusetzen, dass das auf seiner äußeren Oberfläche mit einer zeitlich beschränkten kutanen haftenden Tätowierung (10) ausgestattete Aufbringungskissen (6) befeuchtet wird, wenn ein hinreichender Druck auf den Flüssigkeitsvorrat (3) ausgeübt wird.

2. Vorrichtung nach Anspruch 1, **dadurch gekennzeichnet, dass** die Druckzone (5a) mit dem Vorrat (3) in Verbindung steht und die Ausgangsöffnung des Vorrats (3) bildet, wenn auf letztere ein Druck ausgeübt wird.

3. Vorrichtung nach einem der Ansprüche 1 oder 2, **dadurch gekennzeichnet, dass** der Träger aus mindestens zwei an ihrem Rand durch ein erstes nicht lösbares Verbindungsmittel (11) und durch ein zweites lösbares Verbindungsmittel (12) hermetisch miteinander verbundene Häute (1, 2) gebildet ist, wobei die Haut (2) zwei Vertiefungen aufweist, welche es erlauben, mit der Haut (1) den Flüssigkeitsvorrat (3) und die dichte Zwischenkavität (8) zu definieren.

4. Vorrichtung nach Anspruch 3, **dadurch gekennzeichnet, dass** die Häute (1) und (2) miteinander durch Kleben oder Warmschweißen hermetisch verbunden sind, wobei das Mittel (11) ein nicht lösbares Kleben oder Warmschweißen und das Mittel (12) ein lösbares Kleben oder Warmschweißen ist.

5. Vorrichtung nach einem der Ansprüche 1 bis 4, **dadurch gekennzeichnet, dass** die Öffnung (4) und die dichte Zwischenkavität (8) im Wesentlichen die gleiche Abmessung wie das Aufbringungskissen (6) haben.

6. Vorrichtung nach einem der Ansprüche 1 bis 5, **dadurch gekennzeichnet, dass** das Aufbringungskissen (6), welches hermetisch über seinen gesamten Rand durch ein Verbindungsmittel (7) mit dem Träger verbunden ist, wobei das Verbindungsmittel (7) ein Klebestreifen oder ein endgültiges Warmschweißen ist, die Öffnung (4) gänzlich bedeckt.

7. Vorrichtung nach einem der Ansprüche 1 bis 6, **dadurch gekennzeichnet, dass** das Aufbringungskissen durch ein absorbierendes Material realisiert ist, welches aus der Gruppe ausgewählt ist, welche durch absorbierendes Papier und watteartige oder schwammartige Textilmaterialien gebildet ist.

8. Vorrichtung nach einem der Ansprüche 2 bis 7, **dadurch gekennzeichnet, dass** die Häute (1) und (2) unabhängig voneinander aus einem Kunststoffmaterial oder einem ähnlichen, zusammengesetzten oder metallischen Material sind.

9. Vorrichtung nach den Ansprüchen 7 und 8, **dadurch gekennzeichnet, dass** die Haut (1) ein Aluminium umfassendes zusammengesetztes Material ist, die Haut (2) ein durchsichtiges Copolymer ist und das Aufbringungskissen (6) durch Löschpapier gebildet ist.

10. Vorrichtung nach einem der Ansprüche 1 bis 9, **dadurch gekennzeichnet, dass** der Gang (5) als Abdichtungsmittel (5b) eine zerbrechliche Schweißnaht, welche beschädigt werden oder sich ablösen kann, wenn ein ausreichender Druck auf den Flüssigkeitsvorrat (3) ausgeübt wird, und als Druckzone (5a) eine nicht geschweißte Zone umfasst.

11. Vorrichtung nach einem der Ansprüche 1 bis 10, **dadurch gekennzeichnet, dass** sie einen abziehbaren Schutzfilm umfasst, welcher angepasst ist, den Aufbringungsbausch vollständig zu bedecken und die zeitlich begrenzt haftende kutane Tätowierung zu schützen, wobei der Schutzfilm gegebenenfalls auf seiner Außenseite mit einer Schicht aus einem ablösbaren haftenden Material ausgestattet sein kann.
